(19)

Europäisches
Patentamt
European
Patent Office
Office européen
des brevets

(11) **EP 3 771 699 B1**

(12) **EUROPEAN PATENT SPECIFICATION**

(45) Date of publication and mention
of the grant of the patent:
**25.12.2024 Bulletin 2024/52**

(21) Application number: **19315081.0**

(22) Date of filing: **30.07.2019**

(51) International Patent Classification (IPC):
*C03C 17/34* (2006.01)    *C03C 17/36* (2006.01)
*G16C 20/30* (2019.01)    *G06N 3/12* (2023.01)
*G16C 60/00* (2019.01)

(52) Cooperative Patent Classification (CPC):
**G16C 60/00; G06N 3/126;** C03C 17/3435;
C03C 17/36; C03C 17/361; C03C 17/3626;
C03C 17/3639; C03C 17/3644; C03C 17/3649;
C03C 17/3652; C03C 17/366; G16C 20/30;
G16C 20/70

(54) **COMPUTER IMPLEMENTED METHOD FOR AUTOMATED OPTIMIZATION OF THE PROPERTIES OF EACH LAYER OF A MULTI-LAYERED COATING ON A GLASS SUBSTRATE TO BE PRODUCED IN FUNCTION OF THE DESIRED PROPERTIES FOR SAID MULTI-LAYERED COATING RELATED TO ITS APPLICATION**

COMPUTERIMPLEMENTIERTES VERFAHREN ZUR AUTOMATISIERTEN OPTIMIERUNG DER EIGENSCHAFTEN JEDER SCHICHT EINER MEHRSCHICHTBESCHICHTUNG AUF EINEM HERZUSTELLENDEN GLAS-SUBSTRAT IN ABHÄNGIGKEIT VON DEN GEWÜNSCHTEN EIGENSCHAFTEN DER GENANNTEN MEHRSCHICHTBESCHICHTUNG IN BEZUG AUF IHRE ANWENDUNG

MÉTHODE MISE EN OEUVRE PAR ORDINATEUR POUR L'OPTIMISATION AUTOMATISÉE DES PROPRIÉTÉS DE CHAQUE COUCHE D'UN REVÊTEMENT MULTICOUCHE SUR UN SUBSTRAT DE VERRE À PRODUIRE EN FONCTION DES PROPRIÉTÉS SOUHAITÉES POUR LEDIT REVÊTEMENT MULTICOUCHE EN RAPPORT AVEC SON APPLICATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(43) Date of publication of application:
**03.02.2021 Bulletin 2021/05**

(73) Proprietor: **SAINT-GOBAIN GLASS FRANCE**
**92400 Courbevoie (FR)**

(72) Inventors:
• **Monmeyran, Corentin**
  **75019 Paris (FR)**
• **Hivet, Romain**
  **93400 Saint-Ouen (FR)**

(74) Representative: **Saint-Gobain Recherche**
**41 Quai Lucien Lefranc**
**93300 Aubervilliers (FR)**

(56) References cited:
**EP-B1- 2 804 843        CN-A- 109 239 918**
**JP-A- 2003 315 534**

## Description

The present invention pertains to a computer implemented method for automated optimization of the properties of each layer of a multi-layered coatings on a glass substrate to be produced in function of the desired properties for said multi-layered coating related to its application according to claim 1. The invention aims to cut costs and time in the development and production of new multi-layered coatings, allowing an automated optimization of the properties of each layer of the coatings in function of the desired properties and/or functionalities for the coating.

Multi-layered coatings are used to functionalize surfaces of a wide range of substrates, most of them being transparent substrates such as glass. They can bring optical properties and/or functionalities that suit specific applications.

For instance, in the building industry, surfaces of glass sheets are often coated with multi-layered low-emissivity or solar control coatings. Such coatings are stacks of a plurality of chemically and physically different thin layers which interact with each other in order to modify the incident solar radiation falling on the surfaces of the glass panes. They often contain infrared-reflective metallic layers, mainly made of metals such as silver, gold or copper, to reduce heat and/or radiation transfer through the panes. They are combined with dielectric layers, mainly through sandwiching configurations, in order to counterbalance or adjust colour shifts, reflection and/or transmission effects due to said metallic layers, and to provide the sought solar factor or heat emission rate. Other layers, either metallic of dielectric, can also be used in the coating to improve thermal and/or structural stability over time, during use, or upon subsequent transformations such as heating, tempering and/or bending.

Although multi-layered functional coatings are widely used, the design and development of new stacks of layers remains challenging. For instance, glass panes which will bear such coatings must meet such specific and strict requirements in colours, reflection/transmission rates and thermal durability over time that the chemistry and thickness of each layer and their order in the stack must be finely adjusted. As an example, it is typically the case for colour-on-demand stacks. A customized stack must be designed for each client to meet specific colour requirements. Thus, getting a suitable stack requires the development, the production and the test of a lot of intermediate prototypes which are costly and time consuming.

In order to cut costs and mineral resources, a stack must also be efficient in reducing the amount of rare or precious materials such as silver, titanium, niobium, used in their fabrication. Unfortunately, a newly developed stack which permits to obtain all desired properties for the substrate on which it is deposited can sometimes consume more precious resources than another one, which still has to be developed. Further research and development is then required to find the best compromise between optical/thermal performances and resources savings. Such development is costly and time consuming, and often not compatible with business requirements regarding market timeline. Furthermore, it does not guarantee to find the best solution.

Properties and/or functionalities of stacks can also be improved by the use of new materials or elements as constituents of one or more layers of the coating. However, screening the effects of new materials or elements requires too much investment for sometimes rather unsuccessful results. It would then be advantageous to have a solution which allows to find rather rapidly a group of potential good coating candidates for a given application before producing them as prototypes and picking the best one out from them through performance evaluation campaign.

To get round the issue of developing a new coating for each application, a known alternative is to combine different functional coatings by depositing them on each surface of a substrate or on one or all surfaces of several different substrates used in the same panel. For instance, in building industry, one or more glass sheets comprised in a glazing unit can be coated by different multi-layered functional coatings on one or all of their surfaces. Such solution allows to benefit from the properties of each coating without modification. However, the solar radiation can interact with each functional coating in such unexpected way that the final performances of the whole system can be unsatisfactory for the given application. To prevent such drawback, a screening of the interaction of coatings with each other must be carried out upstream, which is again time and resources consuming. A method, said for instance, that for one functional coating on a first surface of substrate giving satisfactory thermal performances in a glass pane but inappropriate colour, can provide the constituents and physical parameters of another coating which could counterbalance the colours when deposited on the other side of said substrate or on a surface of second substrate to be combined with the first one, would be particularly advantageous for the building industry.

Thus, there is a need for quick screening and design of multi-layered functional coatings in accordance to optical properties and/or functionalities which are looked for in a given coating or coating/substrate system.

EP 1 039 316 A describes a method for producing multi-layered optical filters using a genetic algorithm. The method selects the most optimized combination of refractive index and thickness for each layer in a given stack of layers as optical filter to obtain the desired reflection characteristics with respect to light incident to the filter. However, the method is merely a computer implemented mathematical method which is adapted to optimize only two specific numerical parameters, i.e. refractive index and thickness. It is unable to select other optical properties, such as colours, light reflection and/or transmission, solar factor, low emission, ..., on the whole electromagnetic spectrum of interest for a given application, neither it provides information about the chemistry of each layer.

WO 2006/059788 A describes also a method for producing multi-layered optical filters, but using a distributed genetic algorithm. The method selects the most optimized combination of thickness for each layer in a given stack of layers through iterative steps of migration of randomly selected individuals between groups of individuals, called islands. Between each migration step, a search of local minima of a cost function is performed on each generation computed by a genetic algorithm in each island in order to exclude individuals having poor evaluation. In this method, only one parameter, i.e. thickness of layer, is investigated at once. The chemistry and order of the layers in the stack is fixed by the user. This method is unable to select in same time the chemistry of each layer and other optical properties, such as colours, light reflection and/or transmission, solar factor, low emission, ..., on the whole electromagnetic spectrum of interest for a given application.

JP 2003 315534 A discloses a method of manufacturing an optical multilayer film by means of a genetic algorithm.

The present invention solves the above mentioned problems by providing a method for automated optimization of the properties of each layer of a multi-layered coatings on a glass substrate to be produced in function of the desired properties for said multi-layered coating related to its application, as defined in claim 1. Dependent claims being advantageous embodiments of said method. The invention also pertains to a system and a computer program to implement the method, also defined by the claims. The invention also relates to the use of the method as defined above in a process for fabricating a multi-layered functional coating for a given application.

The method of the invention aims to find the right combination of material and thickness for each layer of the multi-layered functional coating regarding the desired properties for said coating. Hence, it advantageously allows a fast screening of new materials for layers, and a fast investigation of the interaction between different functional specialized coatings which can be used for example, in two-side coated substrates or in units comprising multiple coated substrates like multiple glazing units in the building industry. Furthermore, it allows to reduce time and resources consuming development of intermediate prototypes in the design of new multi-layered functional coating to meet specific requirements for properties while saving mineral resources of precious and/or rare elements by the finding, for each layer, of the most suitable material and the simultaneous optimization of its thicknesses.

In the context of the invention, the following definitions and conventions are used.

Unless stated otherwise or without precision, the word 'thickness' used for a layer or a substrate corresponds to the physical, real or geometrical thickness of said layer or said substrate. It is expressed in nanometres.

A multi-layered functional coating which can be produced by the present invention encompasses all kind of coatings with optical properties. In particular, the coating can be a stack of functional layers and dielectric units, possibly arranged alternatingly.

A functional layer in a coating is a layer which brings, alone or in combination with other layers, optical properties to said coating. In particular, a functional layer can provide function which modifies oncoming electromagnetic radiation to reduce heat and/or radiation transfer. For instance, it can be a radiation-reflective layer in a given wavelength range of the solar spectrum (infrared, visible, ultraviolet...). Typical materials used for metallic functional layer are silver, gold and copper.

The expression 'dielectric unit' refers to one or more layers in contact with each other forming a stack which is overall dielectric, i.e. which does not have the functions of a functional layer. If the dielectric unit comprises more than one layer, those layers can all be themselves dielectric. The physical, real or geometrical thickness of a dielectric unit refers to the sum of the physical, real or geometrical thicknesses of each of the layers that are comprised in said dielectric unit. Typical materials used for layers in dielectric unit are stoichiometric on non-stoichiometric metallic nitrides or oxides like silicon nitride, niobium nitride, silicon oxide, titanium oxide, zinc oxide, tin oxide, tin zinc oxide, alone or in combination.

The noun "stoichiometry" and its derived adjectives are interpreted according to the conventional meaning of the technical field. In particular, it means that the proportions of the chemical elements constituting a compound correspond to those of the "defined compound" as defined by thermochemical diagrams or conventions in said technical field.

Beside functional layer and dielectric units, a multi-layered functional coating which can be produced by the present invention can further comprise auxiliary layers. Examples of auxiliary layers are blocking layers which prevent the migration of chemical species or compounds from one layer to other layer, smoothing layers which affects the roughness and the growing behaviour of layers disposed onto them, and protective layers which acts as a shield against physical and chemical weathering from outside environment. Blocking layers are often disposed beneath or above, close or possibly in contact, to functional layers. Smoothing layers are often disposed beneath, close or possibly in contact, to functional layers. Protective layers are often disposed as the topmost layer of the coating, directly in contact with the outside environment.

When used to qualify a layer, a substrate or a glazing, the word 'transparent' means a layer, a substrate or a glazing through which at least part of the electromagnetic spectrum is transmitted in a useful wavelength range for an intended application so that an object can be distinctly seen through said layer, substrate, or glazing in said useful wavelength range.

In the context of the invention, properties of multi-layered functional coatings encompass all the properties related to their application, their production, and/or the sourcing of materials for their production. They can also encompass the properties or parameters of the coated articles in which said coatings are integrated, such as glazing.

Examples of properties are optical properties, thermal properties, overall costs of the materials to make the coating, and overall deposition rate of the coating in a given deposition process.

In particular, optical properties of multi-layered functional coatings encompass all the properties which polarize, deviate, reflect, select, absorb and/or alter in any other way an incident radiation in the electromagnetic spectrum of interest for a given application. These properties often also influence heat transfers through the coating regarding said incident radiation.

Examples of optical properties are colours, complex and/or real index of refraction, dielectric function, diffuse reflexion, specular reflexion, transmission, angular spectrum, transparency, haziness, ..., on part or whole electromagnetic spectrum of interest for a given application. In particular, for glazing, the electromagnetic spectrum can be advantageously the visible light spectrum, the infrared spectrum, the ultraviolet spectrum, alone or in combination. Another example is the ratio between the quantity of heat from radiation that is transmitted through the coating and the quantity of heat of the incident radiation. In the solar spectrum, this ratio is called solar factor. Another example of optical properties is the transmitted radiation through the coating divided by the previous ratio. In the solar spectrum, this value is called selectivity.

In a multi-layered functional coating, the material for a layer can be characterized by one or several properties. Examples of properties can be:

- the dielectric function of the material on part or whole electromagnetic spectrum of interest for a given application,
- the index of refraction of the material at a given wavenumber or on part or whole electromagnetic spectrum of interest for a given application,
- the deposition rate of the material for a given production method, for instance, chemical vapour deposition (CVD) or physical vapour deposition (PVD) processes,
- the financial costs of the material itself, such as, for instance, the costs of the mineral resources to make said material.

The properties of a multi-layered coating depend on the properties of the material of its layers, so that the latter can be used to calculate the former.

As a non-limitative example, the optical properties of a multi-layered coating can be obtained by calculating the interferences between radiation reflexions and transmissions at each interface between layers forming said coating, said radiation reflexions and transmissions being calculated from the dielectric functions of the materials of layers. The dielectric function of material, also called permittivity, describes the response of said material to the application of an alternating electric field, in particular to the electric field of an electromagnetic radiation. This definition corresponds to the conventional definition in the state of art.

Examples of such calculations are fully described in the state of art, for example in F. Abelès, Le Journal de Physique et le Radium, "La théorie générale des couches minces", 11, 307-310 (1950), or in Born & Wolf, E., Principles of optics: electromagnetic theory of propagation, interference and diffraction of light, Pergamon Press, 1964.

In the context of the present invention, a substrate can be a transparent substrate, like a transparent mineral or organic glass sheet. It can also be a non-transparent, possibly tinted, mineral or organic substrate. The substrate can be flexible or rigid. Examples of materials for organic substrates are polyethylenes, polyesters, polyacrylates, polycarbonates, polyurethanes, polyamides. These polymers can be fluorocarbon-based polymers. Examples of mineral substrates are ceramics, glass-ceramics and glasses, such as sodo-lime glasses, borosilicate glasses, aluminosilicate glasses, alumino-borosilicate glasses.

The present invention involves a genetic algorithm. Genetic algorithms are known as a subcategory of evolution algorithms, i.e. generic population-based metaheuristic optimization algorithms, which mimic the processes of natural selection, in particular processes of mutation, crossover, selection and recombination, in order to find an optimized approximated solution to a given problem without precise knowledge of the fitness landscape of interrelations between parameters of said problem and its output.

Examples of underlying mathematics of genetic algorithm in the field of multi-layered coatings are described in: Martin et al., 'Synthesis of optical multilayer systems using genetic algorithms', Applied Optics, 34:2247-2254, 1995; Patel & Kheraj, 'Optimization of the genetic operators and algorithm parameters for the design of a multilayer anti-reflection coating using the genetic algorithm', Optics & Laser Technology, 70:94-99, 2015; Schubert et al., 'Design of multilayer antireflection coatings made from co-sputtered and low-refractive-index materials by genetic algorithm', Optics Express, 16: 5290-5298, 2008.

For sake of clarity and conciseness, the invention is now detailed by reference to the elements illustrated in the following figures.

[Fig.1] is a schematic representation of a multi-layered functional coatings on a substrate.
[Fig. 2] is an illustrative flowchart of method according to the invention.

[Fig. 3] is a schematic representation of double glazing unit comprising a colour compensating coating.

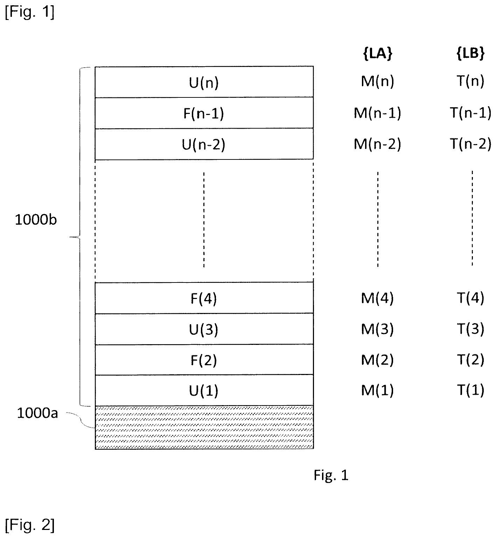

As a non-limitative illustration of the functioning of the invention, an arrangement for a multi-layered functional coating which can be produced by the invention is schematically exemplified on the figure 1. The coating is here represented as a stack 1001b of layers deposited on a substrate 1001a. The layers symbolized U(1), F(2),..., F(n-1), U(n), are alternating layers of functional layers (F) and dielectric units (U). Each layer is made of a given material (M) and has a given thickness (T) which are respectively symbolized in the LA and LB lists.

The substrate 1001a can be a transparent substrate, like a transparent mineral or organic glass sheet. It can also be a non-transparent, possibly tinted, mineral or organic substrate. The functional layers (F) can be radiation-reflective metallic layers, for instance, infrared-reflective metallic layers made of silver. Dielectric units (U) can be formed of one or more layers in contact with each other forming a stack which is overall dielectric. Layers can be made of metallic nitrides and/or oxides, alone or in combination.

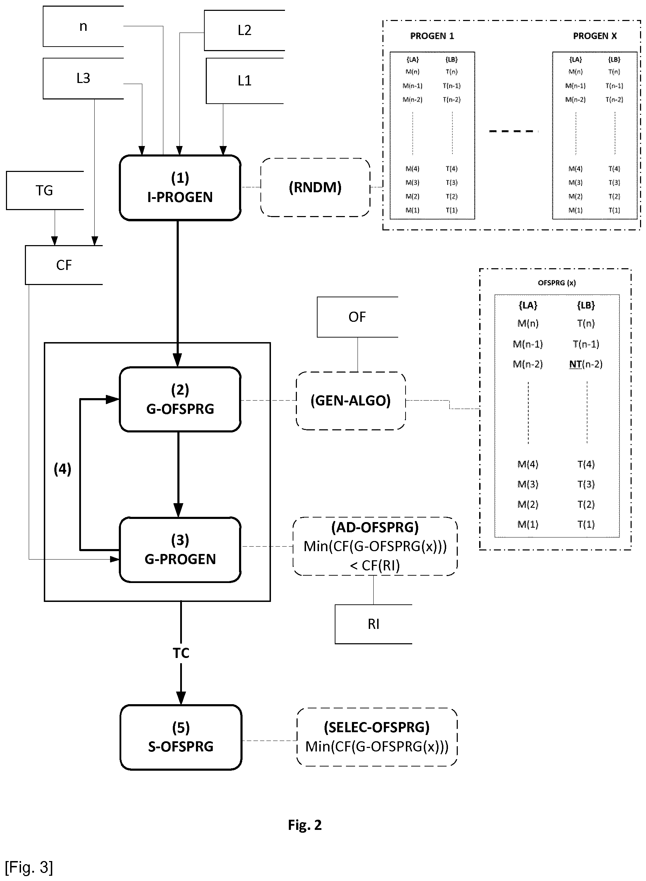

As a matter of clear understanding, the method according to the invention is now detailed by reference to the illustrative flowchart of figure 2.

The flowchart illustrates a computer implemented method for producing a multi-layered functional coating 1001b.

The method takes as inputs

- a number, n, of layers in said coating;
- a list, L1, of materials for the layers of said coating;
- a list, L2, of thickness range for each said material of L1;
- a list, L3, of at least one property of each said material of L1;
- a cost function, CF, based on a single or a combination of properties for the multi-layered functional coating, said properties being calculated from the properties of L3, and targets values, TG, for said optical properties ;
- an optical normalization function, OF, having, as input parameters, thickness and a single or a combination of properties for the multi-layered functional coating, and adapted to return a new value of thickness depending on said input parameters.

The method providing as outputs the material, thickness and order of each layer in the coating along with overall properties of the multi-layered functional coating.

The method comprises the following steps:

(1) an initialisation (I-PROGEN) of a population of a given number, X, of random (RNDM) progenitors (PROGEN1,...PROGEN X), each progenitor being a stack of n layers, said stack of n layers being defined by an ordered list, LA, of n materials selected from L1 for each of the n layers, and by a corresponding ordered list, LB, of n values of thickness for each of the n layers;
(2) a generation (G-OFSPRG) of a population of offspring from the last generated population of progenitors, each offspring being generated from at least two progenitors by a genetic algorithm (GEN-ALGO), said genetic algorithm (GEN-ALGO) comprising a recombination step which replaces at least one value of thickness of the list LB of an offspring by a value (NT) computed by the optical normalization function, OF. On the figure 2, as an example, the thickness of the (n-2) layer of the stack of the offspring OFSPRG(x) is replaced;
(3) a generation (G-PROGEN) of a new population of progenitors (PROGEN) by adding at least an offspring (AD-OFSPRG) which has at least the lowest value for the cost function, CF, comparing to a reference individual, RI, (Min(CF(G-OFSPRG(x))) < CF(RI)) to the last generated population of progenitors;
(4) successive iterations of the steps (2) and (3) until a termination condition, TC, is met;
(5) a selection (S-OFSPRG) of the offspring from the last generated population of offspring, said selected offspring (SELEC-OFSPRG) having the lowest value for the cost function, CF (Min(CF(G-OFSPRG(x)))).

The choice of the given number, X, of progenitors at the step (1) is mainly a matter of computing resources. In particular, it will depend of the complexity of the stack, i.e. the number of layers and nature of the desired properties, of the allowed computation time to perform the method, and of the desired accuracy for the selection of the offspring at step (5). As a rule of thumb, a given number X between, 10 and 1000, preferentially between 20 and 50 can be an advantageous start.

The number, n, of layers given as input to the method of the invention, depends on the properties looked for in the coating. Broadly speaking, at the first time the method of the invention is performed, the number, n, should not be too low as the method may be too much restricted to find a solution. If the case arises, the number, n, can be increased and the method reiterated until a solution is found.

According to certain embodiment of the method of the invention, the at least one property in L3 is selected from the dielectric function, the index of refraction, the deposition for given deposition process, or the financial costs of the material itself.

According to another embodiment of the method of the invention, L3 can be a list of several properties of each material of L1. For instance, a combination of two or several properties selected from the dielectric function, the index of refraction, the deposition for given deposition process, or the financial costs of the material itself.

Depending on the selected at least one property of L3, the properties of the multi-layered coating that can be looked for can be, in particular, one or several properties selected from optical properties, thermal properties, overall costs of the materials to make the coating, and overall deposition rate of the coating in a given deposition process.

As the method, during its execution, removes unnecessary layers in the final coating by setting their thickness to zero, it is more advantageous to provide as input a high number, n, of layers in order to better explore the space of solutions. Thus, in an advantageous embodiment of the method of the invention, the number, n, of layers is comprised between 2 and 50, preferably between 4 and 30, more preferably between 5 and 20. These ranges cover most of the coatings for numerous applications.

A drawback that evolution algorithms, to which genetic algorithms belong to, do not totally avoid when exploring the space of solutions, even if such algorithm score better than many other ones such as gradient algorithms, is the local minima trap. Local minima trap can lead to a solution which solves the problem, but which is not the most optimized.

For example, in the technical field of multi-layered functional coating, a solution for a stack can be found which permits to obtain all desired properties for the substrate on which it is deposited. However, some features of this stack, e.g. thickness, can lead to consume more precious mineral resources than another which still has to be found.

Performing the method independently several times, can help to avoid the local minima trap as the population of progenitors initialised at step (1) is each time different. Same results each time, or most of the times, may suggest a certain confidence in that the most optimised multi-layered functional coating has been found. As a rule of thumb, performing the method 10 to 1000 times, preferentially 25 and 250 times can be advantageous.

Advantageously, the method of the invention also avoids the drawback of the local minima trap, regardless of whether it is performed once or several times, thanks to a recombination step of step (2) which replaces at least one value of thickness of the list LB of an offspring by a value computed by the optical normalization function OC. The optical normalization function is indeed designed to take into account the physics of a multi-layer functional coating, and especially its interferential nature.

As a non-limitative and illustrative example, the optical normalization function, OF, can be advantageously based on a linear combination of thicknesses and complex or real indexes of refraction of the at least two progenitors from which an offspring is generated according to step (2). For instance, an optical normalization function can be for a layer of the offspring:

$$NT = t_{first/second\ progenitor} \left| \frac{n_{first/second\ progenitor}}{n_{second/first\ progenitor}} \right| + \Delta$$

Where $t_{first/second\ progenitor}$ is the thickness of the layer of the first/second progenitor respectively which corresponds to the layer of the offspring, and $n_{first/second\ progenitor}$ and $n_{second/first\ progenitor}$ are respectively the complex or real indexes of refraction of the materials of the layers of the first/second or second/first progenitor which corresponds to the layer of the offspring. $\Delta$ is an arbitrary constant which can be positive, negative or null. This constant is used to introduce much diversity in the change of thickness.

As a variant, $\Delta$ can be a linear function based on a linear combination of thicknesses and complex or real indexes of refraction of the at least two progenitors from which an offspring is generated according to step (2), and of thicknesses and complex or real indexes of refraction of the two other progenitors, eventually randomly selected from the population of progenitors.

Another advantage of the optical normalization function, OF, is that it can also facilitate the change of materials for layers during the generation of offspring by the genetic algorithm, particularly if different kinds of materials are used for the layers of the coating.

Suppose for example that, while the population of offspring is generated at step (2), the material of layer is changed by another one from L1 whereas the thickness is not in the corresponding thickness range of L2 for this another material. Offspring with uncontrolled and unwanted features may be generated, providing unsatisfactory coatings regarding the requirements fixed for the thicknesses of materials through the lists L1 and L2 provided as inputs. This issue can be handled by the optical normalisation function through a change of the value of that thickness by a most suited but not necessarily optimized thickness.

In this scope, in an advantageous embodiment of the invention, the replacement of values in the recombination step of step (2) can be performed if the value for thickness according to optical normalization function OF is comprised in the thickness range of L2 for the corresponding material of L1, otherwise it is replaced by the value of the closest boundary of said thickness range.

The recombination step in step (2) can be performed for one or several layers of one or several offspring. It can be advantageously performed for each layer of one or several offspring.

The recombination step can be carried when a given condition is fulfilled or provided as input to the method. As a non-limitative example of embodiment, the recombination step can be performed only if a number q is below a given recombination rate R and/or matches the order of the layer of the offspring, said number q being a randomly drawn integer below n, the number of layers in said coating.

The recombination rate R is provided as input in the method. It can be a number between 1 and 0 when q is a normalized random number, i.e. a random number between 0 and 1. The lower the recombination rate, the lower the probability that an offspring is affected by a recombination, and reciprocally. The level of the recombination rate depends of the complexity of the stack, i.e. the number of layers and nature of the desired optical properties, of the allowed computation time to perform the method, and of the desired accuracy for the selection of the offspring at step (5). A recombination rate between 0.01 and 0.5, in particular between 0.05 and 0.1 can be reasonable compromise between computational time or workload and accuracy for coating independently of the number of layers it contains.

Introducing some diversity in a generation of offspring can contribute to avoid the local minima and better explore all the possible configuration for the coating while limiting the risk of premature convergence to a not optimized coating. Diversity is an indicator which evaluates how much the offspring, or progenitors, are different to each other. Most common methods to evaluate the diversity is the entropy, the pair-wise Hamming distance, column-based pair-wise distance and column variance distance. Details on measurement methods for diversity can be found, for instance, in the article of Morrison & De Jong, 'Measurement of Population Diversity', International Conference on Artificial Evolution (Evolution Artificielle). Springer, Berlin, Heidelberg, 2001.

Diversity can be introduced through several ways, more specifically by the changing or altering one or several features, i.e. thickness and/or material, of the LA, LB lists of in each offspring.

In an embodiment of the invention, the step (2) can further comprise a step of swapping the $j^{th}$ and $k^{th}$ values in the LA and LB lists respectively of the offspring, j and k being randomly drawn integer below n when a randomly drawn number is below a given switching rate S. This swapping step corresponds to a permutation of the $j^{th}$ and $k^{th}$ values of the LA list, and the $j^{th}$ and $k^{th}$ values in the LB list.

In another embodiment of the invention, the step (2) can further comprise, for each $p^{th}$ layer in the lists LA and LB of the offspring, a step of replacing the $p^{th}$ value of the list LB by a randomly generated value if a randomly generated number is below a given mutation rate M.

In a particular, if said randomly generated number is above the mutation rate M, and if a new randomly generated number is below a crossing rate C, the $p^{th}$ value of the lists LA and LB of the offspring are replaced by the $p^{th}$ value of the lists LA and LB of another progenitor from the population of progenitors, said another progenitor being chosen according to given selection law.

The given selection law for the other progenitor can be a random selection, a lowest value for cost function, or their combination.

In the above described embodiment, the switching rate S, mutation rate, R, and/or the crossing rate, C, are provided as inputs. They can be number between 1 and 0 when normalized random numbers, i.e. random numbers between 0 and 1, are used.

The cost function, CF, at step (3) is based on a single or a combination of properties for the multi-layered coating, said properties being calculated from the properties of L3, and targets values, TG, for said properties. Said properties are preferentially the relevant properties for the given application for which the multi-layered functional coating is intended. The target values are the desired values for said optical properties for said application. In this scope, the cost function CF is constructed as a function evaluating the discrepancy between the properties of an offspring, which represents a potential solution for the sought coating, and the properties of a coating having the desired values for said properties. The way the cost function, CF, performs this evolution is a matter of choice depending on the level of discrepancy that can be tolerated for the application.

The target values can be either discrete values or intervals of values.

According to certain embodiment of the method of the invention, the cost function, CF, can be a sum of squares residuals.

As example, wherein the desired properties are specific colours for the multi-layered functional coating, a convenient cost function can be:

$$CF = \sum_{T,R}(L^*(target) - L^*(offspring))^2 + (a^*(target) - a^*(offspring))^2 + (b^*(target) - b^*(offspring))^2$$

L*a*b* are space parameters of in the CIELAB L*a*b* colour space according to the ISO 11664 standard for light reflexion, R, and/or transmission, T.

As another examples, the cost function, CF, can also be a function of the costs of one or more materials in the lists LA, and/or deposition rates of those material for given deposition process.

Advantageously, the cost function, CF, can also be used to keep a constant size for the population of offspring regarding the size of the population of progenitors by keeping only the same number of those offspring that have the lowest value for cost function. This number often corresponds to the given number, X, of progenitors provided at the step (1).

At step (3) of the method of the invention, the offspring having the lowest value for the cost function, CF, comparing to a reference individual, RI, is added to the population of progenitors.

According to embodiment of the invention, the reference individual can be advantageously one of the at least two progenitors of the population of progenitors which corresponds to the offspring, or the offspring of the population of offspring which has the lowest value for the cost function CF. These reference individuals ensure that at least the best offspring is selected between two generations of the same family made by the offspring and its progenitors, or inside a same generation of different offspring spawned from different progenitors, and this offspring is added to the population of progenitors.

It is also possible, in certain embodiment of the invention, to add more than one offspring whose values of cost function are among the lowest ones comparing to the reference individual. This can potentially help to reduce the computation time of the method without undergoing local minima trap.

In another embodiment of the invention, the reference individual can be randomly selected from a part of populations of progenitors and/or from a part of the population of offspring, said parts comprising those progenitors and/or offspring whose values of cost function are the lowest or are below a given threshold. As a non-limitative example, 20%, preferably 10% of those progenitors and offspring which have the lowest values of cost function can be selected before to randomly select a reference individual among them.

The termination condition of step (4) can be a given maximum number of generations, a criterion for genetic diversity of the generated population of offspring, or an evolution criterion for the average value of cost function for the population of offspring after a given number of iterations.

As a non-limitative illustrative example, the criterion for genetic diversity of the generated population of offspring can be a given value of the pair-wise Hamming distance.

An example of evolution criterion for the average value of cost function for the population of offspring after a given number of iterations can be given percentage of variation of the average value of cost function for the population of offspring after a given number of iterations. If the variation is under this percentage, the iterations of step (4) are stopped. For example, if after five iterations, the average cost function of the population has not decreased by more than 1%, the iterations of step (4) can be stopped.

Alternatively, instead of applying this criterion on the whole population of offspring, it can be applied to part of it, for example, to the part of the population of offspring whose offspring have the best or the worst values for cost function, CF, comparing to a given threshold.

In an embodiment of the invention, the method takes further as input the configuration of system in which the coating is used, said configuration being selected among: single glass unit, double glass unit, triple glass unit, laminated unit. For an implementation in the method, the element of said units, e.g. substrates and additional layers of material or air, can be considered as layers whose optical properties, nature, material and thickness are either fixed as inputs or to be optimized as for the other layers of the coating. Preferentially the optical properties, material, nature and thicknesses of the elements of the units are fixed and provided as inputs to the method, the optical properties of said unit being providing as part of the targets to be obtained for the multi-layered functional coating.

In the context of invention, a single unit is a panel made of only one substrate, eventually transparent. A double unit is a panel made of two substrates, eventually transparent, separated by an air gap. A triple unit is a panel made of three substrates, eventually transparent, separated to each other by two air gaps. A laminated unit is panel made of at least two substrates, eventually transparent, in adhesive contact to each other through an interlayer, eventually a polymeric transparent interlayer. In these units, the substrates can be transparent substrates, like a transparent mineral or organic glass sheet. They can also be non-transparent, possibly tinted, mineral or organic substrates. The substrates can be flexible or rigid.

Another object of the invention is a data processing system comprising means for carrying out the method according to any of the embodiments described herein. Example of means for carrying out the method is a device which can be instructed to carry out sequences of arithmetic or logical operations automatically to perform tasks or actions. Such device, also called computer, can comprise one or more Central Processing Unit (CPU) and at least a controller device that are adapted to perform those operations. It can further comprise other electronic components like input/output interfaces, non-volatile storages devices, and buses that are communication systems for the data transfer between components inside a computer, or between computers. One of the input/output devices can be user interface for human-machine interaction, for example graphical user interface to display human understandable information.

The method of the invention is computer implemented. Another object of the invention is then a computer program comprising instructions which, when the program is executed by a computer, cause the computer to carry out the method of the invention according to any embodiments described herein. Figure 2 provides an example of data flow chart to implement a method for producing a multi-layered functional coating for a given application according to the invention.

Any kind of programming language, either compiled or interpreted, can be used to implement the steps of the method of the invention. The computer program can be part of a software solution, i.e. part of a collection of executable instructions, code, scripts or the like and/or databases.

Another object of the invention is a computer-readable medium comprising instructions which, when executed by a computer, cause the computer to carry out the method according to any of the embodiments described herein.

The computer-readable storage is preferably a non-volatile storage or memory, for example hard disk drive or solid-state drive. The computer-readable storage can be removable storage media or a non-removable storage media as part of a computer.

Alternatively, the computer-readable storage may be a volatile memory inside a removable media. This can ease the deployment of the invention into many production sites.

The computer-readable storage can be part of a computer used as a server from which executable instructions can be downloaded and, when they are executed by a computer, cause the computer to carry out a method according to any of the embodiments described herein.

Alternatively, the instructions can be executed on the server to which client computers can connect and provide data as inputs to the method of the invention. The L1, L2 and L3 lists of the method can be defined by the client computers from databases stored in the server computer. Once data are processed, the output can be downloaded onto the client computer or directly send, for example, as instructions to machines of manufacturing lines of multi-layered functional coating. This kind of implementation can be advantageous as it can be realised in a distributed computing environment such as a cloud computing solution.

The method of the invention can be advantageously used in a process of fabrication of a multi-layered functional coating for a given application. For instance, the output of the method can be provided to process comprising means to fabricate a multi-layered functional coating according to said output.

The method of the invention can also be advantageously directly implemented in a process of fabrication of a multi-layered functional coating for a given application, such as chemical vapour deposition (CVD) or physical vapour deposition (PVD) processes. The process can then be interfaced with the production apparatus of said process so that the method provides directly the instructions to said production apparatus for the production of a multi-layered functional coating.

The advantages of the invention are illustrated by the examples described hereinafter.

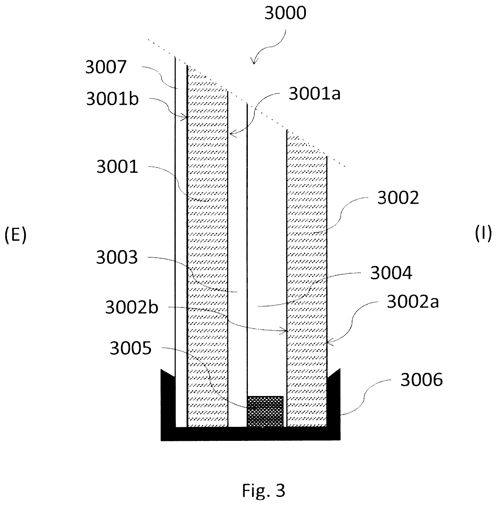

Different functional coatings can be combined by depositing them on each surface of a substrate or on one or all surfaces of several different substrates used in the same glazing. Figure 3 schematically represents a cross-section of an example of a such glazing. The double glazing unit 3000 comprises a first sheet 3001 of mineral glass with an outside surface 3001b and an inside surface 3001a, a second sheet 3002 of mineral glass with an outside surface 3002b and an inside surface 3002a, an insulating gas blade 3004, a spacer 3005 and a seal joint 3006. The mineral glasses are transparent sodalime glasses. On the figure, symbols (E) and (I) are respectively the outside and the inside of the building on whose walls said glazing 3000 can be fixed. A first multi-layered functional internal coating 3003 is deposited on the inside surface 3001a of the first sheet of glass 3001 in contact with the insulating gas blade 3004. This multi-layered functional coating can be, for instance, a low emissive or solar control coating. A second multi-layered functional coating 3007 is deposited on the outside surface 3001b of the first sheet 3001 of glass. This second multi-layered functional coating can be a coating which compensates colour shifts which are due to the first multi-layered functional coating 3003.

In the examples, the method of the invention is used to produce a colour compensating coating 3007 for an internal coating 3001 that already exists in a double glass unit 3000. A coating, C1, is used as said internal coating 3001 and is described in the table 1 below.

| Table 1 | C1 |
| --- | --- |
| Material | Thickness (nm) |
| | Substrate (2001) |
| $TiO_x$ | 9 |
| SnZnO | 7 |
| ZnO | 8,2 |
| Ag | 9.5 |
| NiCr | 0,3 |

(continued)

| Table 1 | C1 |
|---|---|
| **Material** | **Thickness (nm)** |
| | Substrate (2001) |
| ZnO | 6 |
| $Si_3N_4$ | 21 |
| ZnSnO | 11.6 |
| $Si_3N_4$ | 22 |
| ZnSnO | 8 |
| ZnO | 8 |
| Ag | 15 |
| NiCr | 0.6 |
| ZnO | 6 |
| SiZrN | 13 |
| $Si_3N_4$ | 19 |
| ZnSnO | 8.5 |

The optical properties of the outside side (E) of the glazing unit 3000 with the coating C1 as internal coating 3001 but without a compensating coating 3007 are listed in the table 2 below.

| Table 2 | C1 |
|---|---|
| **TL** | 70 |
| **a*T** | -4.5 |
| **b*T** | 3 |
| **L*T** | 87 |
| **Rext** | 13 |
| **a*Rext** | -2 |
| **b*Rext** | -9.5 |
| **L*Rext** | 43 |
| **Rint** | 15 |
| **a*Rint** | 1 |
| **b*Rint** | -1 |
| **L*Rint** | 46 |
| **9** | 37 |
| **s** | 1.9 |

In table 2:

- the light transmission in the visible spectrum, TL, the solar factor, g, and the selectivity, s, and the internal reflection, Rint, and the external reflection, Rext, in the visible spectrum are defined, measured and calculated in conformity with the standards EN 410 and EN 14501. The color is measured in the L*a*b* CIE 1976 chromatic space according to the standard ISO 11664 with a D65 illuminant and a visual field of 2° for the reference observer.
- TL is the value of the light reflection in the visible spectrum, expressed as percentage, measured through the glazing 3000.

- a*T and b*T are the values of the a* and b* parameters measured in transmission at a zero angle of observation with respect to the normal to the surface of the glazing;
- Rext is the value of the light reflection in the visible spectrum, expressed as percentage, measured on the outer surface 3001b of the sheet of soda-lime-silica glass 3001 with a thickness of 6 mm of said double glazing 3000;
- a*Rext and b*Rext are respectively the values of the a* and b* parameters measured on the outer surface 3001b of the sheet of soda-lime-silica glass 3001 with a thickness of 6 mm of the double glazing 3000 along a zero angle of observation with respect to the normal to the surface of the double glazing 3000;
- Rint is the value of the light reflection in the visible spectrum, expressed as percentage, measured on the inner surface 3002a of the sheet of soda-lime-silica glass 3002 with a thickness of 6 mm of the double glazing 3000;
- a*Rint and b*Rint are respectively the values of the a* and b* parameters measured on the inner surface 3002a of the sheet of soda-lime-silica glass 3002 with a thickness of 6 mm of the double glazing 3000 along a zero angle of observation with respect to the normal to the surface of the double glazing 3000.

The compensating coating 3007 has been produced with the method of the invention with

- a number of layers, n = 4;
- a list L1 of materials for layers: NbN, NiCrN, TiN, NiCr, $SiO_2$, $Si_3N_4$, $TiO_2$, SnZnO;
- a list L2 of thickness range for each material of L1:
- a list L3 of optical properties of each said material of L1, said optical properties being the dielectric function of each material and from which the optical properties TL, a*T, b*T, Rext, a*Rext, b*Rext, Rint, a*Rint, b*Rint, g and s can be calculated on the solar spectrum;

The following function has been provided as cost function, CF:

$$CF = \sum_{T,R}(L^*(target) - L^*(offspring))^2 + (a^*(target) - a^*(offspring))^2 + (b^*(target) - b^*(offsrping))^2$$

L*a*b* are space parameters of in the CIELAB L*a*b* colour space according to the ISO 11664 standard for light reflexion, R, and/or transmission, T.

The values for targets in the cost function, CF, are listed in the table 3, Rext and Rint being reflexion from the outside (E) and the inside (I) respectively.

| Table 3 | T | Rext | Rint |
|---------|---|------|------|
| L* | 75 | 52 | 52 |
| a* | -4 | -1 | 0 |
| b* | -2 | -2 | 0 |

The values of parameters a*T, b*T, a*Rext and b*Rext as target values in table 3 are very different from the values of same parameters in table 2.

The following function has been provided to the method as optical normalisation function, OF:

$$NT = t_{first/second\ progenitor}\left|\frac{n_{first/second\ progenitor}}{n_{second/first\ progenitor}}\right| + \Delta$$

Where $t_{first/second\ progenitor}$ is the thickness of the layer of the first or second progenitor respectively which corresponds to the layer of the offspring, and nfirst/second progenitor and Hsecond/first progenitor are respectively the complex or real indexes of refraction of the materials of the layers of the first/second or second/first progenitor which corresponds to the layer of the offspring. Δ is an arbitrary constant.

The method has been implemented so as the replacement of values in the recombination step of step (2) is performed if the value for thickness according to optical normalization function OC is comprised in the thickness range of L2 for the corresponding material of L1, otherwise it is replaced by the value of the closest boundary of said thickness range.

The method has been further implemented so as to use a switching rate S, a mutation rate, M, and a crossing rate, C, as defined in above described embodiments of the invention. In this example, the recombination rate R, the switching rate S, the mutation rate, M, and the crossing rate, C are all fixed to 5%.

The elements of the double glass unit, i.e. the two glass substrates 3001 and 3002, the insulating gas blade 3004 and the internal coating 3003, has been provided as inputs to the method, the optical properties of the double glass unit being providing as part of the targets to be obtained for the multi-layered functional coating.

A given maximum number of generations has been provided as the termination condition, TC. This number was fixed at 100.

The features of the obtained multi-layered functional coating are listed in the table 4 below and the final optical properties of the double glazing 3000 with said obtained coating as colour compensating coating 3007 are listed in the table 5.

| Table 4 | C1 |
|---|---|
| **Material** | **Thickness (nm)** |
| | Substrate (2001) |
| $Si_3N_4$ | 29.4 |
| $SiO_2$ | 23.6 |
| NbN | 2.3 |
| $TiO_2$ | 23.3 |

| Table 5 | DGU |
|---|---|
| **TL** | 48.2 |
| **a*T** | -4.3 |
| **b*T** | -1 |
| **L*T** | 75 |
| **Rext** | 23.9 |
| **a*Rext** | -0.4 |
| **b*Rext** | -1.6 |
| **L*Rext** | 56 |
| **Rint** | 20.0 |
| **a*Rint** | -1.1 |
| **b*Rint** | 0.3 |
| **L*Rint** | 52 |

Comparing the data of tables 2 and 5 to the target values of table 3, it appears that the values of a*T, b*T, a*Rext, b*Rext, L*Rext, a*Rint, b*Rint and L*Rint in the table 5 are much closer to the target values than those in the table 2.

These results clearly show that the method of the invention allows to produce the right combination of materials and thicknesses for the layers of a multi-layered functional coating regarding the desired optical properties sought for said coating used as a compensating coating in the present example.

**Claims**

1. Computer implemented method for automated optimization of the

   properties of each layer of a multi-layered coating on a glass substrate to be produced in function of the desired properties for said multi-layered coating related to its application, said method taking as inputs :

- a number, n, of layers in said coating;
- a list, L1, of materials for the layers of said coating;
- a list, L2, of thickness range for each said material of L1;
- a list, L3, of at least one property of each said material of L1;
- a cost function, CF, based on a single or a combination of properties for the multi-layered coating, said properties being calculated from the properties of L3, and targets values for said properties ;
- an optical normalization function, OF, having, as input parameters, thickness and a single or a combination of desired properties for the multi-layered coating, and adapted to return a new value of thickness depending on said input parameters;

said method providing as outputs the material, thickness and order of each layer in the coating along with properties of the multi-layered coating related to its application;
said method comprising the following steps:

(1) an initialisation of a population of a given number of random progenitors, each progenitor being a stack of n layers, said stack of n layers being defined by an ordered list, LA, of n materials selected from L1 for each of the n layers, and by a corresponding ordered list, LB, of n values of thickness for each of the n layers;
(2) a generation of a population of offspring from the last generated population of progenitors, each offspring being generated from at least two progenitors by a genetic algorithm, said genetic algorithm comprising a recombination step which replaces at least one value of thickness of the list LB of an offspring by a value computed by the optical normalization function OF;
(3) a generation of a new population of progenitors by adding at least an offspring which has at least the lowest value for the cost function, CF, comparing to a reference individual, to the last generated population of progenitors;
(4) successive iterations of the steps (2) and (3) until a termination condition is met;
(5) a selection of the offspring from the last generated population of offspring, said selected offspring having the lowest value for the cost function, CF.

2. Method according to claim 1, wherein the step (2) further comprises a step of swapping the $j^{th}$ and $k^{th}$ values in the LA and LB lists respectively of the offspring, j and k being randomly drawn integer below n when a randomly drawn number is below a given switching rate S.

3. Method according to any of claims 1 to 2, wherein the step (2) further comprises, for each $p^{th}$ layer in the lists LA and LB of the offspring, a step of replacing the $p^{th}$ value of the list LB by a randomly generated value if a randomly generated number is below a given mutation rate M.

4. Method according to claim 3, wherein, if said randomly generated number is above the mutation rate M, and if a new randomly generated number is below a crossing rate C, the $p^{th}$ value of the lists LA and LB of the offspring are replaced by the $p^{th}$ value of the lists LA and LB of another progenitor from the population of progenitors, said another progenitor being chosen according to given selection law.

5. Method according to claim 4, wherein the given selection law for the another progenitor is a random selection, a lowest value for cost function, or their combination.

6. Method according to any of claims 1 to 5, wherein the recombination step in step (2) is performed for each layer of the offspring.

7. Method according to claim 6, wherein the recombination step is performed if a number q is below a given recombination rate R and/or matches the order of the layer of the offspring, said number q being a randomly drawn integer below n.

8. Method according to any of claims 1 to 7, wherein the replacement of values in the recombination step of step (2) is performed if the value for thickness according to optical normalization function OF is comprised in the thickness range of L2 for the corresponding material of L1, otherwise it is replaced by the value of the closest boundary of said thickness range.

9. Method according to any of claims 1 to 8, wherein the reference individual is one of the at least two progenitors of the population of progenitors which corresponds to the offspring, or is the offspring of the population of offspring which has the lowest value for the cost function CF.

10. Method according to any of claims 1 to 9, wherein the termination condition of step (4) is a given maximum number of generations, a criterion for genetic diversity of the generated population of offspring, or an evolution criterion for the average value of cost function for the population of offspring after a given number of iterations.

11. Method according to any of claims 1 to 10, wherein said at least one property in L3 is selected from: the dielectric function, the index of refraction, the deposition for given deposition process, or the financial costs of the material itself.

12. Method according to any of claims 1 to 11, wherein said properties of the multi-layered are selected from: optical properties, thermal properties, overall costs of the materials to make the coating, and overall deposition rate of the coating in a given deposition process.

13. Method according to any of claims 1 to 12, wherein said method takes further as input the configuration of system in which the coating is used, said configuration being selected among: single glass unit, double glass unit, triple glass unit, laminated unit.

14. A computer program comprising instructions which, when the program is executed by a computer, cause the computer to carry out the method according to any of claims 1 to 13.

15. A computer-readable medium comprising instructions which, when executed by a computer, cause the computer to carry out the method according to any of claims 1 to 13.

16. Use of a method according to any of claims 1 to 13 in a process for fabricating a multi-layered functional coating for a given application.


**Patentansprüche**

1. Computerimplementiertes Verfahren für eine automatische Optimierung der Eigenschaften jeder Schicht einer Mehrschichtbeschichtung auf einem Glassubstrat, um in Abhängigkeit von den gewünschten Eigenschaften für die Mehrschichtbeschichtung bezüglich ihrer Anwendung produziert zu werden,

   wobei das Verfahren als Eingaben nimmt:

   - eine Anzahl, n, von Schichten in der Beschichtung;
   - eine Liste, L1, von Materialien für die Schichten der Beschichtung;
   - eine Liste, L2, eines Dickenbereichs für jedes des Materials von L1;
   - eine Liste, L3, von mindestens einer Eigenschaft jedes des Materials von L1;
   - eine Kostenfunktion, CF, basierend auf einer einzelnen oder einer Kombination von Eigenschaften für die mehrschichtigen Beschichtung, wobei die Eigenschaften aus den Eigenschaften von L3 berechnet werden, und Zielwerten für die Eigenschaften;
   - eine optische Normalisierungsfunktion, OF, die, als Eingabeparameter, eine Dicke und eine einzelne oder eine Kombination von gewünschten Eigenschaften für die Mehrschichtbeschichtung aufweist und angepasst ist, um abhängig von den Eingabeparametern einen neuen Dickenwert zurückgeben;

   wobei das Verfahren als Ausgaben das Material, die Dicke und die Reihenfolge jeder Schicht in der Beschichtung zusammen mit den Eigenschaften der Mehrschichtbeschichtung bezüglich ihrer Anwendung bereitstellt;
   das Verfahren umfassend die folgenden Schritte:

   (1) eine Initialisierung einer Population einer gegebenen Anzahl von zufälligen Vorläufern, wobei jeder Vorläufer ein Stapel aus n Schichten ist, der Stapel aus n Schichten durch eine geordnete Liste, LA, von n Materialien, die aus L1 für jede der n Schichten ausgewählt werden, und durch eine entsprechende geordnete Liste, LB, von n Dickenwerten für jede der n Schichten definiert ist;
   (2) eine Generierung einer Population eines Nachkommen aus der zuletzt generierten Population von Vorläufern, wobei jeder Nachkomme durch einen genetischen Algorithmus aus mindestens zwei Vorläufern generiert wird, der genetische Algorithmus umfassend einen Rekombinationsschritt, der mindestens einen Dickenwert der Liste LB eines Nachkommen durch einen Wert ersetzt, der durch die optische Normalisierungsfunktion OF berechnet wird;

(3) eine Generierung einer neuen Population von Vorläuferzellen durch Hinzufügen von mindestens einem Nachkommen, der im Vergleich zu einem Referenzindividuum mindestens den niedrigsten Wert für die Kostenfunktion, CF, zu der zuletzt generierten Population von Vorläuferzellen aufweist;

(4) aufeinanderfolgende Iterationen der Schritte (2) und (3), bis eine Abbruchbedingung erfüllt ist;

(5) eine Auswahl des Nachkommen aus der zuletzt generierten Population des Nachkommen, wobei der ausgewählten Nachkomme den niedrigsten Wert für die Kostenfunktion, CF, aufweist.

2. Verfahren nach Anspruch 1, wobei der Schritt (2) ferner einen Schritt eines Austauschens des j-ten und k-ten Werts in der LA- beziehungsweise der LB-Liste des Nachkommen umfasst, wobei j und k zufällig gezogene ganze Zahlen unter n sind, wenn eine zufällig gezogene Zahl unter einer gegebenen Wechselrate S liegt.

3. Verfahren nach einem der Ansprüche 1 bis 2, wobei der Schritt (2) ferner, für jede p-te Schicht in den Listen LA und LB des Nachkommen, ein Schritt des Ersetzens des p-ten Werts der Liste LB durch einen zufällig generierten Wert umfasst, falls eine zufällig generierte Zahl unter einer gegebenen Mutationsrate M liegt.

4. Verfahren nach Anspruch 3, wobei, falls die zufällig generierte Zahl über der Mutationsrate M liegt und falls eine neue zufällig generierte Zahl unter einer Kreuzungsrate C liegt, der p-te Wert der Listen LA und LB des Nachkommen durch die p-te Wert der Listen LA und LB eines anderen Vorläufers aus der Population der Vorläufer ersetzt wird, wobei der andere Vorläufer gemäß einem gegebenen Auswahlgesetz ausgesucht wurde.

5. Verfahren nach Anspruch 4, wobei das gegebene Auswahlgesetz für den anderen Vorläufer eine zufällige Auswahl, eines niedrigsten Werts für die Kostenfunktion oder ihre Kombination ist.

6. Verfahren nach einem der Ansprüche 1 bis 5, wobei der Rekombinationsschritt in Schritt (2) für jede Schicht des Nachkommen durchgeführt wird.

7. Verfahren nach Anspruch 6, wobei der Rekombinationsschritt durchgeführt wird, falls eine Zahl q unter einer gegebenen Rekombinationsrate R liegt und/oder mit der Reihenfolge der Schicht des Nachkommen übereinstimmt, wobei die Zahl q eine zufällig gezogene ganze Zahl unter n ist.

8. Verfahren nach einem der Ansprüche 1 bis 7, wobei der Ersatz der Werte in dem Rekombinationsschritt von Schritt (2) durchgeführt wird, falls der Wert für die Dicke gemäß der optischen Normalisierungsfunktion OF in dem Dickenbereich von L2 für das entsprechende Material von L1 enthalten ist, andernfalls er durch den Wert der nächstgelegenen Grenze des Dickenbereichs ersetzt wird.

9. Verfahren nach einem der Ansprüche 1 bis 8, wobei das Referenzindividuum einer der mindestens zwei Vorläufer der Population von Vorläufern ist, die dem Nachkommen entspricht, oder der Nachkomme der Population des Nachkommen ist, der den niedrigsten Wert für die Kostenfunktion CF aufweist.

10. Verfahren nach einem der Ansprüche 1 bis 9, wobei die Abbruchbedingung von Schritt (4) eine gegebene maximale Anzahl von Generationen, ein Kriterium für eine genetische Vielfalt der generierten Population des Nachkommen oder ein Evolutionskriterium für den Durchschnittswert der Kostenfunktion für die Population des Nachkommen nach einer gegebenen Anzahl von Iterationen ist.

11. Verfahren nach einem der Ansprüche 1 bis 10, wobei die mindestens eine Eigenschaft in L3 ausgewählt wird aus: der dielektrischen Funktion, dem Brechungsindex, der Abscheidung für einen gegebenen Abscheidungsprozess oder den finanziellen Kosten des Materials selbst.

12. Verfahren nach einem der Ansprüche 1 bis 11, wobei die Eigenschaften der Mehrschichtstruktur ausgewählt werden aus: optischen Eigenschaften, thermischen Eigenschaften, Gesamtkosten der Materialien, um die Beschichtung herzustellen, und Gesamtabscheidungsrate der Beschichtung in einem gegebenen Abscheidungsprozess.

13. Verfahren nach einem der Ansprüche 1 bis 12, wobei das Verfahren ferner als Eingabe die Konfiguration des Systems nimmt, in dem die Beschichtung verwendet wird, wobei die Konfiguration ausgewählt wird aus: Einfachglaseinheit, Doppelglaseinheit, Dreifachglaseinheit, Verbundglaseinheit.

14. Computerprogramm, umfassend Anweisungen, die, wenn das Programm durch einen Computer ausgeführt wird, den Computer veranlassen, das Verfahren nach einem der Ansprüche 1 bis 13 vorzunehmen.

15. Computerlesbares Medium, umfassend Anweisungen, die, wenn sie durch einen Computer ausgeführt werden, den Computer veranlassen, das Verfahren nach einem der Ansprüche 1 bis 13 vorzunehmen.

16. Verwendung eines Verfahrens nach einem der Ansprüche 1 bis 13 in einem Prozess zum Fertigen einer Mehrschichtfunktionsbeschichtung für eine gegebene Anwendung.

**Revendications**

1. Procédé implémenté par ordinateur destiné à l'optimisation automatisée des propriétés de chaque couche d'un revêtement multicouche sur un substrat en verre à produire en fonction des propriétés souhaitées pour ledit revêtement multicouche, apparentées à son application,

   ledit procédé prenant en guise d'entrées :

   - un nombre, n, de couches dans ledit revêtement ;
   - une liste, L1, de matériaux pour les couches dudit revêtement ;
   - une liste, L2, de plages d'épaisseur pour chaque matériau précité de L1 ;
   - une liste, L3, d'au moins une propriété de chaque matériau précité de L1 ;
   - une fonction de coût, CF, en fonction d'une unique propriété ou d'une combinaison de propriétés pour le revêtement multicouche, lesdites propriétés étant calculées à partir des propriétés de L3, et de valeurs cibles pour lesdites propriétés ;
   - une fonction de normalisation optique, OF, ayant, en guise de paramètres d'entrée, l'épaisseur et une unique propriété souhaitée ou une combinaison de propriétés souhaitées pour le revêtement multicouche, et conçue pour renvoyer une nouvelle valeur d'épaisseur en dépendance desdits paramètres d'entrée ;

   ledit procédé fournissant en guise de sorties le matériau, l'épaisseur et l'ordre de chaque couche dans le revêtement en même temps que les propriétés du revêtement multicouche, apparentées à son application ;
   ledit procédé comprenant les étapes suivantes :

   (1) une initialisation d'une population d'un nombre donné de progéniteurs aléatoires, chaque progéniteur étant une pile de n couches, ladite pile de n couches étant définie par une liste ordonnée, LA, de n matériaux choisis parmi L1 pour chacune des n couches, et par une liste ordonnée correspondante, LB, de n valeurs d'épaisseur pour chacune des n couches ;
   (2) une génération d'une population de descendants à partir de la dernière population générée de progéniteurs, chaque descendant étant généré à partir d'au moins deux progéniteurs par un algorithme génétique, ledit algorithme génétique comprenant une étape de recombinaison qui remplace au moins une valeur d'épaisseur de la liste LB d'un descendant par une valeur calculée par la fonction de normalisation optique OF ;
   (3) une génération d'une nouvelle population de progéniteurs en ajoutant au moins un descendant qui a au moins la valeur la plus basse pour la fonction de coût, CF, par comparaison avec un sujet de référence, à la dernière population générée de progéniteurs ;
   (4) des itérations successives des étapes (2) et (3) jusqu'à ce qu'une condition d'achèvement soit respectée ;
   (5) une sélection du descendant parmi la dernière population générée de descendants, ledit descendant sélectionné ayant la valeur la plus basse pour la fonction de coût, CF.

2. Procédé selon la revendication 1, dans lequel l'étape (2) comprend en outre une étape de permutation des $j^{\text{ème}}$ et $k^{\text{ème}}$ valeurs dans les listes LA et LB respectivement du descendant, j et k étant un nombre entier tiré au hasard inférieur à n lorsqu'un nombre tiré au hasard est inférieur à un taux de commutation S donné.

3. Procédé selon l'une quelconque des revendications 1 à 2, dans lequel l'étape (2) comprend en outre, pour chaque $p^{\text{ème}}$ couche dans les listes LA et LB du descendant, une étape de remplacement de la $p^{\text{ème}}$ valeur de la liste LB par une valeur générée au hasard si un nombre généré au hasard est inférieur à un taux de mutation M donné.

4. Procédé selon la revendication 3, dans lequel, si ledit nombre généré au hasard est supérieur au taux de mutation M, et si un nouveau nombre généré au hasard est inférieur à un taux de franchissement C, la $p^{\text{ème}}$ valeur des listes LA et LB du descendant est remplacée par la p<sup>ème</sup> valeur des listes LA et LB d'un autre progéniteur parmi la population de progéniteurs, cet autre progéniteur étant choisi selon une loi de sélection donnée.

5. Procédé selon la revendication 4, dans lequel la loi de sélection donnée pour cet autre progéniteur est une sélection au hasard, une valeur la plus basse pour la fonction de coût, ou leur combinaison.

6. Procédé selon l'une quelconque des revendications 1 à 5, dans lequel l'étape de recombinaison à l'étape (2) est mise en oeuvre pour chaque couche du descendant.

7. Procédé selon la revendication 6, dans lequel l'étape de recombinaison est mise en oeuvre si un nombre q est inférieur à un taux de recombinaison R donné et/ou concorde avec l'ordre de la couche du descendant, ledit nombre q étant un nombre entier tiré au hasard inférieur à n.

8. Procédé selon l'une quelconque des revendications 1 à 7, dans lequel le remplacement de valeurs dans l'étape de recombinaison de l'étape (2) est mis en oeuvre si la valeur pour l'épaisseur selon la fonction de normalisation optique OF est comprise dans la plage d'épaisseur de L2 pour le matériau correspondant de L1, sinon elle est remplacée par la valeur de la limite la plus proche de ladite plage d'épaisseur.

9. Procédé selon l'une quelconque des revendications 1 à 8, dans lequel le sujet de référence est l'un parmi les au moins deux progéniteurs de la population de progéniteurs qui correspond au descendant, ou est le descendant de la population de descendants qui a la valeur la plus basse pour la fonction de coût CF.

10. Procédé selon l'une quelconque des revendications 1 à 9, dans lequel la condition d'achèvement de l'étape (4) est un nombre maximal donné de générations, un critère pour une diversité génétique de la population générée de descendants, ou un critère d'évolution pour la valeur moyenne de la fonction de coût pour la population de descendants après un nombre donné d'itérations.

11. Procédé selon l'une quelconque des revendications 1 à 10, dans lequel ladite au moins une propriété dans L3 est choisie parmi : la fonction diélectrique, l'indice de réfraction, le dépôt pour un processus de dépôt donné, ou les coûts financiers du matériau lui-même.

12. Procédé selon l'une quelconque des revendications 1 à 11, dans lequel lesdites propriétés du multicouche sont sélectionnées parmi : propriétés optiques, propriété thermique, coûts globaux des matériaux pour réaliser le revêtement et taux de dépôt global du revêtement dans un processus de dépôt donné.

13. Procédé selon l'une quelconque des revendications 1 à 12, dans lequel ledit procédé prend en outre en guise d'entrée la configuration d'un système dans lequel le revêtement est utilisé, ladite configuration étant sélectionnée parmi : unité de verre simple, unité de verre double, unité de verre triple, unité stratifiée.

14. Programme d'ordinateur comprenant des instructions qui, lorsque le programme est exécuté par un ordinateur, amènent l'ordinateur à effectuer le procédé selon l'une quelconque des revendications 1 à 13.

15. Support lisible par ordinateur comprenant des instructions qui, lorsqu'elles sont exécutées par un ordinateur, amènent l'ordinateur à effectuer le procédé selon l'une quelconque des revendications 1 à 13.

16. Utilisation d'un procédé selon l'une quelconque des revendications 1 à 13 dans un processus permettant de fabriquer un revêtement fonctionnel multicouche pour une application donnée.

[Fig. 1]

|  | {LA} | {LB} |
|---|---|---|
| U(n) | M(n) | T(n) |
| F(n-1) | M(n-1) | T(n-1) |
| U(n-2) | M(n-2) | T(n-2) |
| ⋮ | ⋮ | ⋮ |
| F(4) | M(4) | T(4) |
| U(3) | M(3) | T(3) |
| F(2) | M(2) | T(2) |
| U(1) | M(1) | T(1) |

1000b

1000a

Fig. 1

[Fig. 2]

**Fig. 2**

[Fig. 3]

Fig. 3

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- EP 1039316 A
- WO 2006059788 A

- JP 2003315534 A


**Non-patent literature cited in the description**

- **F. ABELÈS**. La théorie générale des couches minces. *Le Journal de Physique et le Radium*, 1950, vol. 11, 307-310
- Principles of optics: electromagnetic theory of propagation. **BORN** ; **WOLF, E.** interference and diffraction of light. Pergamon Press, 1964
- **MARTIN et al.** Synthesis of optical multilayer systems using genetic algorithms. *Applied Optics*, 1995, vol. 34, 2247-2254

- **PATEL** ; **KHERAJ**. Optimization of the genetic operators and algorithm parameters for the design of a multilayer anti-reflection coating using the genetic algorithm. *Optics & Laser Technology*, 2015, vol. 70, 94-99
- **SCHUBERT et al.** Design of multilayer antireflection coatings made from co-sputtered and low-refractive-index materials by genetic algorithm. *Optics Express*, 2008, vol. 16, 5290-5298
- Measurement of Population Diversity. **MORRISON** ; **DE JONG**. International Conference on Artificial Evolution. Springer, 2001